# EUROPEAN PATENT APPLICATION

(11) **EP 3 485 874 A1**
(43) Date of publication of application: **22.05.2019**
(21) Application number: 17202221.2
(22) Date of filing: 17.11.2017
(51) Int. Cl.: A61K 9/08, A61K 31/00, A61K 47/12, A61K 47/10, A61K 47/26, A61K 47/34

(54) **METHOD OF PREPARING A NATURAL DEEP EUTECTIC SOLVENT (NADES)-ACTIVE INGREDIENT SYSTEM; A NADES-, AND PREFERABLY A POLYMERIC PRECIPITATION INHIBITOR CONTAINING NADES-, BASED DRUG FORMULATION TECHNOLOGY AND DEVELOPMENT METHOD**

(71) Applicant: SeraNovo B.V., 2312 NV Leiden (NL)
(72) Inventor: Kluft, Bastiaan, 2312 NV Leiden (NL); Hodgins, Niall, 2312 NV Leiden (NL)
(74) Representative: V.O.

(57) **Abstract**

The present invention relates to a method of preparing or optimizing a system comprising a natural deep eutectic solvent (NADES) and active ingredients. Preferably, such systems include a polymeric precipitation inhibitor (PPI). Further the invention relates to formulations comprising or consisting of such developed NADES based systems.

## Description

The present invention relates to a method of preparing or optimizing a NADES (Natural Deep Eutectic Solvent)-active ingredient system; that is, at least one type of an active ingredient solubilized in a NADES. Furthermore the invention relates to a formulation technology composed of a natural deep eutectic solvent (NADES), one or more active ingredients and preferably one or more polymeric precipitation inhibitors (PPI). The NADES is specifically designed and the PPI is specifically selected for one or more active ingredients. In addition the invention relates to the inclusion of one or more type of polymeric precipitation inhibitors. The liquid formulations are suitable for oral or rectal administration. Furthermore the systems are suitable for intravenous or intermuscular administration.

Active ingredients, such as drugs, flavors, fragrances, agrochemicals, dyes *etc.,* both from synthetic and natural origin or sources, are often poorly soluble in water. In practice, the dealing with such active ingredients involves all kinds of extraction, purification, administration steps, and these steps require the use of less polar solvents, such as alcohols, acetone, ethyl acetate, chloroform *etc.* Such solvents present several problems such as: toxicity for the producer/patient/consumer, environmental problems, explosions and the like.

Ionic liquids can be environmentally benign replacements for the traditional volatile organic solvents in various chemical processes. These ionic liquid solvents have in general a negligible vapor pressure and are hence also considered to be safe solvents. That is, their lack of volatility greatly reduces any chance of exposure other than by direct physical contact with skin or by ingestion. However, most conventional ionic liquids are irritating and have a toxicity comparable to common organic solvents. From biological tests it appeared that the toxicity of ionic liquids is mainly determined by the type of cation and that ionic liquids with short alkyl substituents in the cation usually have a lower toxicity.

In WO 2011/155829, a process is described that is based on the finding that some specific naturally occurring materials can suitably be used for extracting materials from biological sources. These materials are deep eutectic solvents (or mixtures) of natural origin or ionic liquids of natural origin.

Deep eutectic solvents are liquids having a melting point that is much lower than the melting points of the two compounds that form the eutectic mixture. Generally, they are formed between a variety of quaternary ammonium salts and carboxylic acids. The deep eutectic phenomenon was first described in 2003 for a mixture of choline chloride and urea in a 1:2 mole ratio, respectively. Other deep eutectic solvents of choline chloride are formed with phenol and glycerol. Deep eutectic solvents are able to dissolve many metal salts like lithium chloride and copper(II)oxide. Also, organic compounds such as benzoic acid and cellulose have great solubility in deep eutectic solvents. Compared to ordinary solvents, eutectic solvents have a very low volatility and are non-flammable. They share a lot of characteristics with ionic liquids, but they are ionic mixtures and not ionic compounds.

Instead, choline citrate is a real ionic liquid. This compound 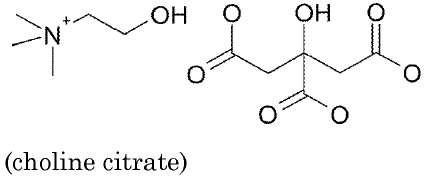 was formed by dissolving citric acid in water, followed by addition of choline hydroxide (in the ratio 2:1) dissolved in methanol. The solvent (water and methanol) was evaporated. The product choline citrate was a slightly yellow viscous liquid, and not a solid. This is probably the first naturally occurring ionic liquid found.

In addition to the ions, sugar-based liquids can be deep eutectic solvents.

According to WO 2011/155829 a process for extracting materials from biological material is provided, which process is characterized in that the naturally occurring biological material is treated with an extractant consisting of a deep eutectic solvent of natural origin or an ionic liquid of natural origin to produce a biological extract of natural origin dissolved in the said solvent or ionic liquid.

These deep eutectic solvents of natural origin and natural ionic liquids are suitable extractants for biological materials. These extractants are very efficient and selective, and as they are of natural origin, they are extremely efficient and suitable for extracting components from biological materials, resulting in an efficient process, providing a good yield. The melting points of the deep eutectic mixtures and ionic liquids is preferable below 25°C. The materials are thus preferably liquid at ambient temperatures.

In Analytica Chimica Acta 766 (2013) 61-68, Dai *et al.* describe their study of a number of deep eutectic solvents and ionic liquids made from a number of natural products. These are used to replace harsh organic solvents and have been applied to many chemical processing steps such as extraction and synthesis applications. Particularly, they describe that they discovered that many plant abundant primary metabolites changed their state from solid to liquid when they were mixed in proper ratio. This finding made them hypothesize that natural deep eutectic solvents (NADES) play a role as alternative media to water in living organisms. They additionally tested a wide range of natural products, and this resulted in the discovery of over 100 NADES from nature. They also measured some physical properties of the NADES such as water activity, density, viscosity, polarity and thermal properties.

In a number of publications, studies of specific NADES systems are described. For example, Rozema et al., in RSC Adv., 2015, 5, 61398-61401, report on the feasibility and benefits of dissolving the poorly water-soluble salsalate in a NADES composed of 1,2-propanediol-choline-water; Faggian et al., in Molecules 2016, 21, 1531, describe a study on the water solubility of rutin in the presence of a number of NADES prepared from sugars, amino acids and organic acids; and Duarte et al., in the Eur. J. Pharmaceutics and Biopharmaceutics 114 (2017) 296-304, describe therapeutic deep eutectic solvents based on menthol complexed with ibuprofen, benzoic acid and phenyl acetic acid.

These publications all start from specific NADES which are used with a particular active ingredient.

In a first aspect, the present invention aims at providing a method for preparing or optimizing a NADES-active ingredient system. That is, dependent on a particular active ingredient and especially an active pharmaceutical ingredient, a suitable NADES is designed, rather than starting from a particular NADES and trying to use that with a particular active ingredient.

That is, in an attempt to overcome the problem of low solubility and therefore low bioavailability (such as in the Biopharmaceutics Classification System (BCS) Class II compounds; information related to the BCS system can be found on the FDA website (https://www.fda.gov/aboutfda/centersoffices/officeofmedicalproductsandtoba cco/cder/ucm128219.htm)) of active ingredients in for example the gastrointestinal tract without needing unnecessarily high dosages of the active ingredient to be used and hence without potential toxicity issues or other side effects of said active ingredient, an optimized NADES is searched for.
Said in other words, in the first aspect the invention aims at formulating a suitable NADES for a specific active ingredient, which will also be referred to herein-below as "target compound", and which will often be a small molecule drug.

Hence, in a first aspect, the invention relates to a method of preparing or optimizing a NADES-active ingredient system, comprising the following steps:
(a) analyzing the active ingredient or target compound;
(b) matching the active ingredient to at least one potential NADES starting composition;
(c) synthesizing a stable, liquid NADES-active ingredient system; and
(d) optimizing the system prepared in step (c).

In a very preferred embodiment, as subsequent step, step
(e) polymeric precipitation inhibitor selection and optimization, is carried out.

The method of the invention hence starts with a particular target compound, which will in practice have a poor water solubility. In the first step of the method, said compound is analyzed for a number of criteria.

This analysis at least involves determining or otherwise providing or collecting the solubility of the active ingredient(s) in a number of solvents, ranging from highly polar to very nonpolar, for example a polar solvent such as water, a moderately polar solvent such as ethanol or DMSO and a nonpolar solvent such as hexane or mineral oil. These values are expressed in mg/ml and determined at a temperature of 25°C at 1 atm. In a preferred embodiment, also the solubility is determined in water-based systems having different pH's, or having different temperatures; and in water-based systems wherein co-solvents are present; and in stomach or intestinal media. Solubility information in a broad range of solvents (including oils, dichloromethane, methanol), is useful because the knowledge of in which type of solvents the active ingredient dissolves well, helps in designing a NADES with similar properties.

Further, the analysis also at least involves determination of acidity/basicity of the compound by determining the pKa value by standard methods. In the case of previously analyzed compounds such information typically available in published literature.

Moreover, the analysis also requires determination of the general structure of the active ingredient, inclusive of determination of the functional groups. Since NADES form, for instance, hydrogen bonds with the active ingredient, an approximate idea of the solubility possibilities can be made by determining which functional groups are able to participate in the desired interaction.

In a preferred embodiment, step (a) involves determining the solubility of the active ingredient or target compound in a number of solvents, ranging from highly polar to very nonpolar, for example a polar solvent such as water, a moderately polar solvent such as ethanol or DMSO and a nonpolar solvent such as hexane or mineral oil; involves determining the pKa value of the active ingredient or target compound; and determining or providing the general structure of the active ingredient of target compound.

In a preferred embodiment, also the dose of the active ingredient that is desired to be administered to an individual in need thereof may play a role in finding a suitable NADES.

In addition to the criteria listed in the previous paragraphs, secondary criteria are helpful in finding a suitable NADES, too. These secondary criteria, each on themselves or in any combination with each other, relate to preferred or special embodiments.

Suitable secondary criteria are for instance the absolute oral bioavailability, typically denoted as a percentage of the maximum bioavailability of 100% which is defined as the bioavailability of the intravenous formulation as measured in a living being such as those used in animal trials or in human trials. Bioavailability is measured as the AUC (Area Under Curve) of the plasma concentration of a drug collected over a certain period of time, which depends on the half-life of the drug. Another suitable criterion is the stability data (such as temperature sensibility and pH range of stability expressed in time units). This is expressed as a duration in days/months/years where the compound has not undergone unacceptable levels of degradation (<10%) in specific conditions e.g. low pH.

Yet, a further secondary criteria is based on the biopharmaceutics classification system (BCS) classification, expressed in classes where for instance Class II refers to compounds that are poorly water soluble but highly permeable. A compound is considered poorly water soluble if the highest dose strength is insoluble in 250 ml water over a pH range of 1 to 7.5. A compound is considered highly permeable when the extent of absorption in humans is determined to be > 90% of an administered dose, based on mass-balance or in comparison to an intravenous reference dose. This information is pertinent to compound selection where technology used to improve solubility may not have any effect of permeability; thus bioavailability may not improve despite improved solubility.

Yet, a further secondary criteria is based on the possibility of ionization. The degree to which a compound can be ionized is related to the pKa; typically the further away from a neutral pKa the greater the possibility of ionizing the compound (salt formation). Also, additional factors can affect the formation such as the practicality of forming the salt. If there is too much steric hindrance around the ionizing group or if the energy required to create the salt is damaging to the structural integrity of the molecule, this can be prohibitive. Further, cross-checks with similar components in built data-bases, can be used.

Derivable from the solubility in various (organic) solvents is for example the polarity of the active ingredient, and this polarity plays an important role in selecting NADES constituents. A high solubility in for example methanol gives an indication that more polar NADES may be successful.

In a preferred embodiment, the method of the present invention hence also comprises that in step (a) at least one of the following parameters is determined from the group consisting of the oral bioavailability, stability, solubility in a range of solvents, the BCS classification, and the possibility of ionization.

For many active ingredients, the analysis does not need to be done from scratch. For many active ingredients, the solubility in various solvents and the pH information or pKa values are known. Pharmacopeia, for instance, mention a lot of data for active pharmaceutical ingredients.

In addition, NADES work through the forming of a hydrogen bonding network. Groups that are good at forming hydrogen bonds are for example =O, OH, NHx, N=R, conjugated systems, acidic/basic groups and in general electronegative groups (F, O, Cl, N, Br, I, S, C, H) with a dipole moment. Oxygen groups in rigid formations such as esters and ethers are not very good at forming hydrogens bond, neither are amides. Freedom of free electron pairs can play a large role in the success of formation. It is especially worth noting that benzene or other aromatic ring structures and more in general compounds having double bonds usually dissolve well in NADES and have some hydrogen bonding capability.

Functional groups also give an indication, in combination with pH stability and solubility, to the possibility of acidic/basic instability and possible solubility increase due to protonation.

After the compound analysis, in method step (b) potential NADES are formulated based on the data gathered in step (a). That is, at least one potential NADES starting composition defined, which will subsequently be optimized in steps (c) and (d). The information on the chemical architecture of the target compound gathered in step (a) hence also assists in finding suitable candidates to make suitable NADES. Dependent on specifications such as low toxicity (preferably the constituents of a NADES are GRAS (Generally Regarded As Safe) certified or have an LD₅₀ value of for example >5000 mg/kg), natural occurrence (*e.g*., sugars, metabolites, organic acids and bases, amino acids), common and accepted use in drug formulations (e.g., propylene glycol), and/or low cost, constituents for the NADES to be prepared are selected.

Subsequently, selected constituents are weighed out in the appropriate molar ratios and corresponding masses and are added to a suitable receptacle, such as a small bottle or other small vessel. In the receptacle generally a stirring element is added, such as a stirring flea. If two or more solid constituents are present, these are generally heated, for instance to about 70°C. When at least one liquid constituent is present, it is generally not needed to heat the system to temperatures higher than about 50°C. The constituents are stirred. When two (or more) solid constituents are mixed, this often leads to high or very high viscosities, in which case addition of some water or other liquid excipients such as propylene glycol is recommended. The stirring is continued until a clear liquid is formed.

The NADES to be designed in the method of the invention is a eutectic mixture composed of one or more of the following compound classes, organic acids, organic bases, sugars, glycols, amino acids, choline or choline derivatives, such as phosphatidyl choline.

In a preferred embodiment, the NADES contains an organic acid which may be one of, but not limited to, malic acid, maleic acid, citric acid, lactic acid and acetic acid.

In another embodiment, the NADES contains an organic base, which may be one of, but not limited to, urea, acetamide, guanine or nicotinamide.

Further, the NADES may contain at least one sugar selected from the group consisting of, but not limited to, sucrose, glucose, fructose, lactose, maltose, xylose, sucrose, inositol, xylitol and ribitol, as well as their phosphates.

The NADES may additionally contain at least one amino acid. Suitable amino acids may be selected from, but are not limited to, for example alanine, glutamic acid, glutamate, asparagine, aspartic acid, lysine, arginine, proline and threonine.

In a further embodiment of the invention, the NADES can contain a glycol. Such a glycol can *e.g.* be selected from, but is not limited to, propylene glycol, butylene glycol, ethylene glycol, glycerol and polyglycerol.

In a preferred embodiment, the optimization step (d) comprises that at least one of the following steps are carried out:
(i) variation of the molar ratios of the constituents;
(ii) addition of a further constituent;
(iii) tuning the pH;
(iv) variation of the water content; and/or
(v) inclusion of solubilisers.

It goes without saying that on the basis of many experiments and/or many data, such as data gathered from (scientific) article sources, an extensive database may be composed. In this database, information on solubility values for many compounds in many different NADES formulations (in mg/ml); a large range of NADES constituents, with molar ratios, water content, formulation stability (*e.g*. at room temperature (RT)) as well as other formulation information; pH/pKa/pKb values (of the NADES); dilutions; temperature details and method of formation information; polarity; viscosity information; *etc.* may be stored.

In this way, a database containing a large number of NADES with accompanying characteristics can be obtained, facilitating the matching step (b).

It is worth noting that NADES do not follow regular solubility principles in many ways, for example the Hansen Solubility Parameters fail immensely in predicting any solubility in NADES. The special hydrogen bonding network that is created brings interactions to the table that cannot be easily predicted by simple modelling. This makes a linear approach very complicated, as values for polarity and pH cannot accurately predict the solubility. An example would be a lactic acid based NADES, which is a great solubiliser of non-polar compounds (as a NADES) but is a highly polar liquid.

Based on the information collected, for example suitably stored in a database, the experience obtained and continuous new formulations of NADES that can be added to the database, a first selection of a number, such as 5, 9 or 12, NADES candidates are made and these NADES are synthesized, first without active ingredient and further tests may be done.

After a clear liquid is obtained, it is preferred to let it cool and sit for 24 hours or so, as a check for stability. If a NADES is not stable at RT (room temperature; in other words, if the eutectic point is higher than RT at the selected molar ratio), one can try adding additional constituents or trying a different molar ratio. If the present inventors do not attain a stable, clear liquid at RT, the NADES is deemed not to be a suitable system.

Once cooled, the pH of the system is measured. An acidic NADES will in general be good at solubilizing a basic active ingredient due to protonation and *vice versa.* The stability can also be influenced by the pH, where usually a range of pH values is given that results in the best stability.

In case crystals are formed in the NADES, which can occur naturally after a certain period of time, adding one or two parts of water may be useful. When trying to solubilize compounds that are poorly soluble in water, adding water may however reduce the solubility of the compound in the NADES. To solve this problem low polarity liquids, such as 1,3- butylene glycol, which is safe and of relatively low viscosity, may be used.

The present inventors have found that visual inspection of the solubility results is a very powerful tool. Usually it is apparent within an hour whether or not a solvent is going to achieve any worthwhile solubility value. If the solute sticks to the side of the receptacle and/or floats on top, investigation into this solvent can be terminated. If the solute makes for a cloudy solution, further dilution may yield success.

The number of NADES prepared are ranked for their solubility. Depending on a comparison of the characteristics, the most successful constituents are identified.

As said, depending on what characteristics the solubility of a given target compound seems to be dependent on, or which avenues are to be investigated further, a new set of NADES is synthesized. If a particular NADES performed well, several new ones are formulated using that NADES as scaffold according to the methods used in step d. Several methods can be investigated to tune the formulation:
- Vary molar ratios of the constituents (more polar constituents, more non-polar constituents);
- Add a further constituent, depending on the properties that were more effective determinants of solubility (add an acid if low pH showed higher efficacy, add a non-polar constituent if less polar NADES had higher efficacy etc.);
- Tune the pH, possible with neutral NADES to add a small amount of HCl or NaOH and monitor the pH;
- Vary the water content;
- Include solubilisers which are also NADES constituents e.g. nicotinamide or propylene glycol.

The optimisation and dilution phases are a recursive loop that are repeated until the objectives are achieved.

When a NADES is diluted with water or introduced in an aqueous system, such as intestinal fluid as in a body, the solvent breaks up and the active ingredient precipitates out of the solution. This precipitation may prevent the active ingredient from being absorbed, due to the poor dissolution profile of large, crystalline drug particles. In order to prevent increase in size of the precipitate of active ingredient particles and to promote an amorphous particulate state, polymeric precipitation inhibitors (PPIs) may be added to the mixture. These PPIs inhibit crystal growth by associating with the drug particles and also stabilize the supersaturated state that occurs when the higher concentration of active ingredient in the NADES is introduced into an aqueous environment where the solubility is significantly less. The maximum concentration in the aqueous environment of the active ingredient is typically several orders of magnitude lower than the maximum concentration in the designed NADES. The PPIs used, increase the solubility in two distinct ways through a metastable state, where up to several orders of magnitude increased solubility can be attained, as well as smaller sizes of precipitant and lower rates of crystal growth are observed over a period of time up to several hours.

That is, following the completion of the aforementioned stages (a to d), stage e is preferably initiated to select the optimum PPI or PPI combination to be included in the formulation.

In this embodiment, following the creation of an appropriate NADES-active ingredient system, the optimum PPI is selected. PPI's are analysed for solubility in the aforementioned system, and initial PPI's are selected for testing based upon predicted strong interactions with the active ingredient. A PPI to active ingredient ratio of approximately 1:1 by weight is analysed for solubility, according to the following steps:
- An appropriate mass of PPI is weighed into a sample (of say 20 ml) of the NADES, designed in steps a - d, at RT.
- The NADES and PPI are stirred at *e.g.* 2000 RPM for 5-10 minutes using a high RPM stirrer in a suitable receptacle.
- The mixture is left to rest for up to 3 days.
- Solubility is inspected visually, whereby a transparent, homogenous solution is considered fully solubilized.
- If the solution is not transparent and homogeneous the mixture is diluted 2 fold with NADES. The stirring and resting process is repeated.

All PPI's that are soluble at approximately a PPI to active ingredient ratio of 1 to 5 are selected for the next stage of testing. A small sample of for example 100 µl is taken from the NADES:PPI mixture and the appropriate mass of active ingredient is solubilized in the system as above. Each NADES: PPI: active ingredient sample is diluted approximately 100 fold with intestinal medium solutions e.g. Fast State Simulated Intestinal Fluid (FaSSIF). The system with the smallest particle size over a 2 hour period is determined to be the best performing formulation. The particle size is determined using visual inspection and optical microscopy.

The aforementioned PPI selection method may be repeated with a second PPI or surfactant to improve the final particle size. The final formulation may be composed as follows NADES: PPI₁: PPI₂/surfactant: active ingredient.

Particularly, these PPIs as used in the formulation to prevent growth of large precipitant crystals upon dilution. The active ingredient may still precipitate, but the crystals are sufficiently small such that these would still be absorbed in the intestine.

The precipitant molecules should in this light have a size less than approximately 10/µm, preferably less than 5µm and ideally less than about 1µm. These sizes are measured at 10 minute intervals over a 2 hours period to analyse the crystal growth. The crystal sizes are monitored using an optical microscope in initial samples; yet are more accurately measured after 2 hours using a Malvern Mastersizer 3000 or similar particle sizing equipment.

Suitable PPI's are crystal growth inhibiting polymers, preferably having a low toxicity. Such polymers may be selected from the groups consisting of, but not limited to, polyvinylpyrrolidones and cellulosic polymers. Suitable cellulosic polymers encompass sodium carboxymethyl cellulose, hydroxypropylmethyl cellulose, methyl cellulose, hydroxypropyl cellulose and ethyl cellulose among others.

The polymers are suitably used in a ratio of polymer to active ingredient between 0.1:1 and 10:1, and preferably between 0.5:1 and 5:1.

In one embodiment, the polymer used is mixed with the NADES-active ingredient mixture and stirred at approximately 2000RPM for 5 minutes and then left to rest. The solubilizing process for polymers may be slow and may take several days to fully solubilize. In another embodiment, the polymer used is solubilized in a diluting solution e.g. FaSSIF intestinal medium and subsequently heated and stirred at approximately 30-60°C. A suitable stirring rate is about 50 r.p.m.

This use of PPIs is specifically effective for BCS class II compounds. Such compounds suffer from a low bioavailability due to poor solubility in water, but have no problems in permeating the intestinal membranes. A capsule containing NADES, one or more active ingredient and one or more PPI's can be specifically tuned to dissolve and release its contents in the duodenum of the intestines, and possibly with conventional tablets that dissolve at a particular pH (5-6 for the duodenum). Once released, the supersaturated state will be effective for at least approximately half an hour, and preferably an hour or longer, greatly increasing the absorption due to the several orders of magnitude increase in solubility. The PPI may be incorporated in the capsule coating, and thus be present in the intestinal fluid upon release of the active ingredient and thus inhibit the precipitation and crystallization process.

This invention allows delivery of a water insoluble active ingredients to an *in vivo* target via oral administration (*e.g.* as a drink or capsule) or via injection.
The invention will be described in further detail in the following nonlimiting examples. In these examples, reference will be made to Figures, wherein
Fig. 1: shows a microscope photograph of pure flufenamic acid at 10x magnification. Particularly, 1 mg flufenamic acid is mixed with 1 ml pure water over 2 hours after which the photograph was taken;
Fig. 2: shows a microscope photograph of flufenamic acid at 40x magnification. 28 mg/ml flufenamic acid is solubilized in choline chloride: propylene glycol (1:3) with 14 mg/ml Polyethylene Glycol-4000 solubilized in Fasted State Simulated Intestinal Fluid (FaSSIF) intestinal medium (poor result);
Fig. 3: shows a microscope photograph of flufenamic acid at 40x magnification. 28 mg/ml flufenamic acid is solubilized in choline chloride: propylene glycol (1:3) with 56 mg/ml Polyethylene Glycol-4000 solubilized in FaSSIF intestinal medium (good result); and
Fig. 4: shows a microscope photograph of paclitaxel at 40x magnification. 30 mg/ml paclitaxel is solubilized in choline chloride: propylene glycol (1:3) with 30 mg/ml hypromellose acetate succinate solubilized in FaSSIF intestinal medium (good result).

### Example - 1 - Flufenamic Acid

Flufenamic acid is a poorly water-soluble molecule, and the intention was to design a system using the aforementioned invention. The goal of the project was to design a NADES with a high solubility for flufenamic acid. Water solubility = 9*10⁻³ mg/ml

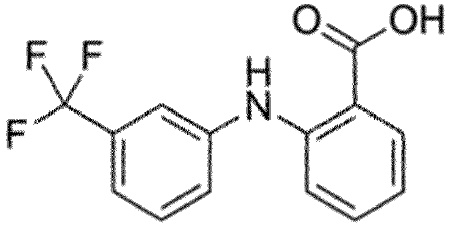

### Stage 1

### Solubility of Flufenamic acid is comparable solvents

Flufenamic acid has a very low water solubility, thus it is expected that the solubility in highly polar solvents will be low. Furthermore the solubility in mineral oil (paraffin oil), hexane and cyclohexane is also very low, thus it is expected that non-polar solvents will be low.
Comparatively the solubility of flufenamic in ethanol and dimethyl sulfoxide is very high (>50mg/ml). This indicates that NADES of medium polarity will be effective solubilizers of flufenamic acid.

### pKa of Flufenamic acid

Flufenamic acid has a pKa of 3.65. A low pKa indicates the compound is readily de-protonated and thus acidic in nature. Acidic compounds typically solubilize better in basic solvents, thus the pH of the designed NADES should be greater than 7.

### Structure

The presence of a carboxylic acid group present in the flufenamic structure coupled with the lack of basic groups indicates the compound will be acidic in nature. The carboxylic acid is also the only very polar group in the molecule. However the presence of conjugated ring structures indicates extra hydrogen bonding possibilities. Fluor-containing groups also have a tendency towards hydrogen bonding, though a CF₃ group exhibits this in low amounts due to the limited polarity shift.

### Stability

Flufenamic acid is stable in both acidic and basic aqueous solutions over a period of 3 months at RT. Flufenamic acid is a highly stable molecule so no additional considerations concerning pH or storage need to be taken into account.

### Suitability of NADES constituents.

Based on the solubility of flufenamic acid in ethanol and DMSO, coupled with the pKa and structural information the NADES should minimally be neutral and ideally basic and moderately non-polar. Suitable constituents could include basic constituents such as choline chloride, nicotinamide, urea and acetamide. Neutral constituents such as propylene glycol, butylene glycol and glycerol.

Due to the poor solubility in polar solvents such as water, polar constituents should be discarded such as sugars (e.g. glucose, fructose and sucrose) sugar alcohols (e.g. sorbitol and xylitol), carboxylic acids (e.g. lactic acid, malic acid and citric acid) and other polar molecules e.g. urea and water.

### Solubility of flufenamic acid in various solvents

Polar NADES
Sorbitol: Urea (1:1)- <1mg/ml - pH 8.3
Choline chloride: Lactic acid: water (2:1:2) - <1mg/ml - pH 2.19
Choline chloride: glucose: butylene glycol (2:1:1) - <1mg/ml

### Neutral NADES

Choline Chloride: Propylene Glycol (1:3) - 28mg/ml - pH6.71
Choline Chloride: Ethylene Glycol (1:3) - 28mg/ml - pH 6.08

### Basic NADES

Acetamide: Propylene Glycol (1:3) - 50mg/ml - pH 6.89
Acetamide: Propylene Glycol (1:2) - 93.1mg/ml - pH 6.47

### Particle Size data

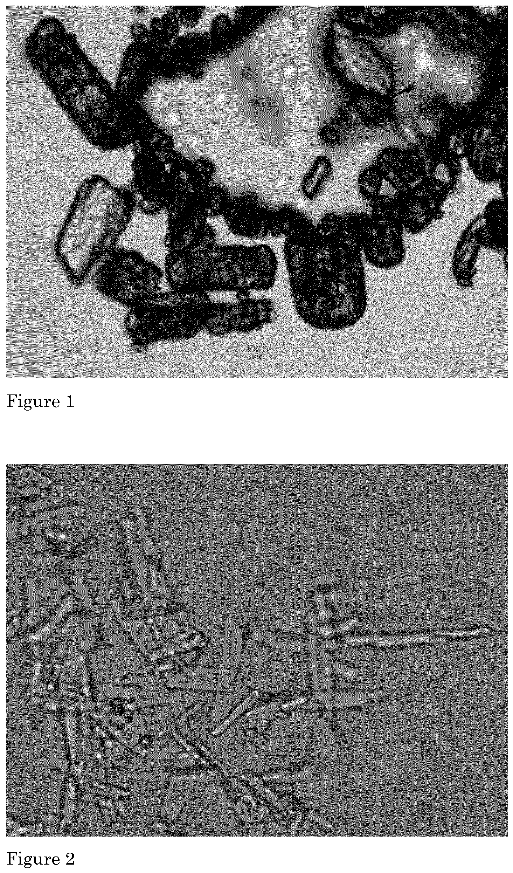

Below are microscope images of formulations of flufenamic acid diluted with intestinal medium. The dilution is approximately similar to 1ml of formulation being released into the intestine. The goal is to have sufficiently small particle sizes for a sufficiently long period of time to promote drug absorption. Figures 1, 2 and 3 show progressions in particle size of flufenamic acid in formulations designed using the present invention.

### Example 2 - Paclitaxel

The following example is of a poorly water-soluble molecule, Paclitaxel, and the process of designing a system using the aforementioned invention. The objective of the project was to have design a NADES able to solubilize a high concentration of paclitaxel. The second objective was to select one or more PPI's capable of controlling the size of precipitant upon an approximately 100 fold dilution with intestinal medium. The target dose of the formulation was 30mg/ml. The variation in particle size between formulations was analysed with visual inspection. The best performing formulations were compared with a size calibrated optical microscope and camera.
Paclitaxel has a water solubility of 0.006mg/ml at 25°C and 1atm.

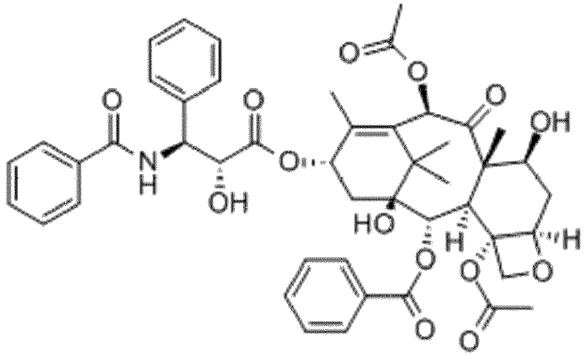

### Stage 1 - Solvent design

Paclitaxel is partially soluble in ethanol (1.5mg/ml)
Paclitaxel has a pKa of 11.9

### Solubility of Paclitaxel is comparable solvents

Paclitaxel has a water solubility of 0.006mg/ml, this indicates it is unlikely to be soluble in highly polar NADES. Solvents of slightly lower polarity such as ethanol have a paclitaxel solubility of 1.5mg/ml. Low polarity solvents such as caster oil and olive oil perform better with approximate solubility values of 15-20mg/ml.
This information indicates that when designing a NADES high polarity constituents should be discarded.

### pKa of Paclitaxel

Paclitaxel has a pKa of 11.9, this value indicates it is a difficult compound to protonate/de-protonate, thus it is unlikely to have increased solubility in strong acids or strong bases.
This information indicates that when designing a NADES, constituents with a pH lower than 3 or higher than 9 will not have an added effect through and may be counterproductive since acids/based usually have increased polarity versus neutral compounds.

### Structure

Paclitaxel has a complicated ring structure which shields many of the more polar oxygen-containing groups such as hydroxyls and ketones, which are present in low to moderate quantities. Combined with the lack of protonatable groups this gives a plausible explanation for the very poor aqueous solubility of paclitaxel. The existence of large hydrophobic areas indicates the need to reproduce these in a suitable solvent. The presence of conjugated ring structures increases the feasibility of using NADES, which are able to induce hydrogen-bonding like behaviour with the π-orbitals of these conjugated systems.

### Suitability of NADES constituents

Due to the low solubility of paclitaxel in highly polar solvents e.g. water, high polarity NADES constituents should be discarded e.g. sugars (Glucose, fructose and sucrose), sugar alcohols (sorbitol and xylitol) and carboxylic acids (citric acid, lactic acid and malic acid).
Due to the pKa of almost 12 solvents of pH less than 3 and higher than 9 should be excluded e.g. citric acid and urea. This is combined with additional stability considerations, which show that Paclitaxel is most stable between pH 3-6 and degrades quickly at basic pH (8). NADES constituents of low to moderate polarity (e.g. butylene glycol) and a pH between 4 and 7 (e.g. choline chloride) should be included.

### Solubility of paclitaxel in NADES

### Polar NADES

Sucrose: Glucose: Fructose: Water (1:1:1:8) - <1mg/ml - pH 3.36
Choline Chloride: Urea: Water (1:1:2) - <1mg/ml - pH 9.80

### Moderate polarity and pH NADES

Choline Chloride: Propylene Glycol (1:3) - 30mg/ml - pH 6.71

### Acidic NADES

Malic acid: Beta alanine: Water (1:1:2) - <1mg/ml - pH 3.43

### Dose

Choline Chloride: Propylene Glycol at a molar ratio of 1:3 achieved the desired dose of 30mg/ml. Based on the structure and hydrogen bond forming potential of paclitaxel cellulosic polymeric precipitation inhibitors were selected to be analysed the formulations PPI. Cellulosic polymers contain large hydrophobic domains intertwined with hydroxyl groups capable of hydrogen bonding, which match the structure very well. This is in contrast with for example PVP (Polyvinylpyrollidone) polymers which do not contain strong hydrogen bonding groups.
According to the methods mentioned in the invention 8 cellulosic PPI's were solubilized in the designed NADES at ratios of PPI's to active ingredient between 0.5 and 5 to 1.

Hypromellose Acetate Succinate was the best performing PPI at a PPI to active ingredient ratio of 1:1. Optical microscopy results pictured below show the size of observable particles.

### Particle Size Data

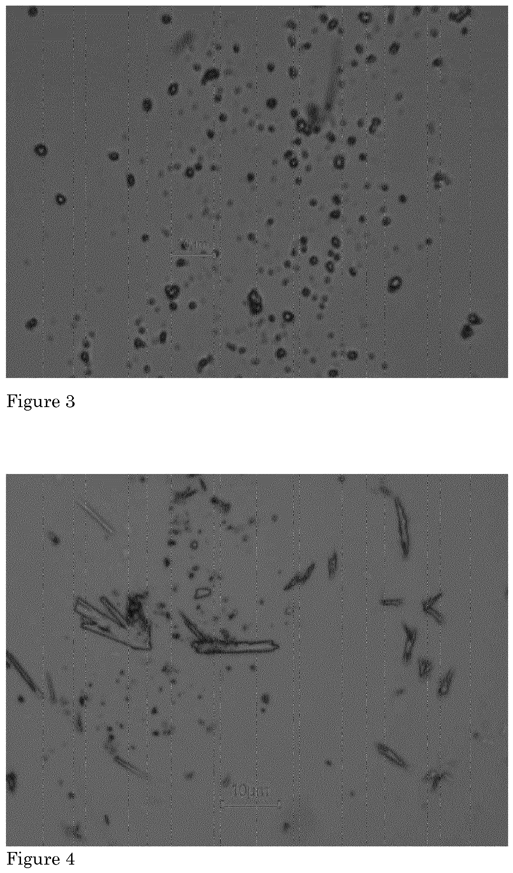

Below is a microscope image of a formulation of paclitaxel diluted with intestinal medium. The dilution is approximately similar to 1ml of formulation being released into the intestine. Figures 4 shows particles of paclitaxel, the majority of which are less than 10 µm. Particles less than 1 µm are not detected by this optical microscopy method. Only the largest paclitaxel particles are visible in this image.

## Claims

1. A method of preparing or optimizing a system comprising a NADES and one or more active ingredients, comprising the following steps:
(a) analyzing an active ingredient or target compound;
(b) matching the active ingredient to at least one potential NADES starting composition;
(c) synthesizing a stable, liquid NADES-active ingredient system; and
(d) optimizing the system prepared in step (c).

2. The method of claim 1, further comprising step
(e) selecting a polymeric precipitation inhibitor (PPI) and optimizing the system.

3. The method of claim 1 or claim 2, wherein in step (a) involves determining the solubility of the active ingredient or target compound in a number of solvents, preferably ranging from highly polar to very nonpolar, for example a polar solvent such as water, a moderately polar solvent such as ethanol or DMSO and a nonpolar solvent such as hexane or mineral oil; involves determining the pKa value of the active ingredient or target compound; and involves determining or providing the general structure of the active ingredient of target compound.

4. The method of any one of claims 1-3, wherein in step (a) further at least one of the following parameters is determined from the group consisting of the oral bioavailability, stability, solubility is a range of solvents, the BCS classification, and the possibility of ionization.

5. The method according to any one of the preceding claims, wherein at least one of the following steps are carried out:
(i) variation of the molar ratios of the constituents;
(ii) addition of a further constituent;
(iii) tuning the pH;
(iv) variation of the water content; and/or
(v) inclusion of solubilisers.

6. The method of any one of the preceding claims, further comprising the addition of one or more surfactants to the NADES.

7. The method according to any one of claims 2 and claims dependent on claim 2, wherein the PPI has low toxicity to the human body, preferably an LD50 value in humans of >2500mg/kg, and more preferably >5000mg/kg.

8. The method according to any one of claims 2 and claims dependent on claim 2, wherein the PPI may be selected from the group consisting of and preferably from the group consisting of polyvinylpyrrolidones and cellulosic PPI's; cellulosic PPI's encompassing sodium carboxymethyl cellulose, hydroxypropylmethyl cellulose, methyl cellulose, hydroxypropyl cellulose and ethyl cellulose.

9. The method according to any one of claims 2 and claims dependent on claim 2, wherein the PPI to active ingredient ratio is between 0.1: 10 and 10:1 and preferably between 0.5:1 and 5:1.

10. The method according to any one of claims 2 and claims dependent on claim 2, wherein the PPI is selected based on a minimum solubility in the NADES of 10% of the mass of the solubilized active ingredient.

11. The method according to any one of claims 2 and claims dependent on claim 2, wherein the PPI is selected based on the active ingredient precipitant particle size for a 100 fold dilution of the system in intestinal medium.

12. The method according to any one of claims 6 and claims dependent on claim 6, wherein the surfactant is pharmaceutically acceptable, and preferably selected from a glycerol ester, a sorbitan ester, a sorbitol ester, a polyglycerol ester, a fatty alcohol, a propylene glycol ester, an alkyl glucoside ester, a sugar ester, lecithin, a silicone (co)polymer and mixtures thereof.

13. A formulation comprising, or consisting of, a NADES obtainable by the method of any one of claims 1-12, one or more active ingredients, and, optionally, one or more PPIs.

14. The formulation according to claim 13, wherein the size of active ingredient precipitant upon dilution in aqueous environments is less than 50 µm, preferably less than 10 µm, more preferably less than 5 µm and ideally less than 1 µm.

15. The formulation according to claim 13 or 14, wherein the system is deliverable orally or rectally optionally via a capsule; or deliverable intravenously or intramuscularly, optionally via injection.
